(19) 

Europäisches
Patentamt
European
Patent Office
Office européen
des brevets

(11) **EP 4 042 944 A1**

(12) **EUROPEAN PATENT APPLICATION**
published in accordance with Art. 153(4) EPC

(43) Date of publication:
**17.08.2022 Bulletin 2022/33**

(21) Application number: **20871331.3**

(22) Date of filing: **25.09.2020**

(51) International Patent Classification (IPC):
***A61B 8/12*** (2006.01)

(52) Cooperative Patent Classification (CPC):
**A61B 8/12**

(86) International application number:
**PCT/JP2020/036452**

(87) International publication number:
**WO 2021/065750 (08.04.2021 Gazette 2021/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.09.2019 JP 2019178983**

(71) Applicant: **TERUMO Kabushiki Kaisha
Tokyo 151-0072 (JP)**

(72) Inventors:
• **SAKAMOTO, Yasukazu
Ashigarakami-gun, Kanagawa 259-0151 (JP)**
• **SHIMIZU, Katsuhiko
Ashigarakami-gun, Kanagawa 259-0151 (JP)**

• **ISHIHARA, Hiroyuki
Ashigarakami-gun, Kanagawa 259-0151 (JP)**
• **JACQUET, Clément
Sakai-shi, Osaka 599-8231 (JP)**
• **HENN, Thomas
Sakai-shi, Osaka 599-8231 (JP)**
• **HERATH, Nuwan
Sakai-shi, Osaka 599-8231 (JP)**
• **ERIKSSEN, Iselin
Sakai-shi, Osaka 599-8231 (JP)**
• **SAGA, Ryosuke
Sakai-shi, Osaka 599-8231 (JP)**

(74) Representative: **Casalonga
Casalonga & Partners
Bayerstraße 71/73
80335 München (DE)**

(54) **DIAGNOSIS ASSISTANCE DEVICE, DIAGNOSIS ASSISTANCE SYSTEM, AND DIAGNOSIS
ASSISTANCE METHOD**

(57) This diagnosis assistance device generates three-dimensional data of a biological tissue on the basis of tomographic data of the biological tissue into which a catheter has been inserted and displays the generated three-dimensional data on a display as a three-dimensional image, the diagnosis assistance device comprising a control unit that specifies a point on the biological tissue with which a tip of the catheter is in contact in the three-dimensional data as a contact point, and sets the color of the voxel corresponding to the contact point to a predetermined color in the three-dimensional image.

*FIG. 3*

EP 4 042 944 A1

**Description**

Technical Field

The present disclosure relates to a diagnostic assistance device, a diagnostic assistance system, and a diagnostic assistance method.

Background Art

PTL 1 to PTL 3 disclose a technique of generating a three-dimensional image of a cardiac cavity or a blood vessel using a US image system. The term "US" is an abbreviation of ultrasound.

Citation list

Patent Literature

PTL 1: U.S. Patent Application Publication No. 2010/0215238
PTL 2: US Patent No. 6385332
PTL 3: US Patent No. 6251072

Summary of Invention

Technical Problem

Treatment using IVUS is widely performed on a cardiac cavity, a cardiac blood vessel, a lower limb artery region, and the like. The term "IVUS" is an abbreviation of intravascular ultrasound. The IVUS is a device or a method for providing a two-dimensional image of a plane perpendicular to a long axis of a catheter.

At present, an operator needs to perform treatment while reconstructing a three-dimensional structure by stacking two-dimensional images of IVUS in his/her brain, which is a barrier particularly to young doctors or inexperienced doctors. In order to remove such a barrier, it is conceivable to automatically generate a three-dimensional image representing a structure of a biological tissue such as a cardiac cavity or a blood vessel from the two-dimensional images of IVUS and display the generated three-dimensional image toward the operator. When a catheter other than an IVUS catheter, such as an ablation catheter, is inserted into the biological tissue, it is conceivable to further display a three-dimensional image representing the other catheter.

However, in the three-dimensional image, it may be difficult to confirm whether the catheter and the biological tissue are in contact with each other.

An object of the present disclosure is to facilitate confirming whether a catheter and a biological tissue are in contact with each other in a three-dimensional image.

Solution to Problem

A diagnostic assistance device according to an aspect of the present disclosure is a diagnostic assistance device configured to generate three-dimensional data of a biological tissue inserted with a catheter based on tomographic data of the biological tissue, and display the generated three-dimensional data as a three-dimensional image on a display. The diagnostic assistance device includes: a control unit configured to specify a point of the biological tissue with which a distal end of the catheter is in contact as a contact point in the three-dimensional data, and set a color of a voxel corresponding to the contact point to a predetermined color in the three-dimensional image.

In one embodiment, the control unit is configured to adjust a ratio for including an element of the predetermined color in a color of each voxel of the three-dimensional image in accordance with a distance from the distal end of the catheter to each point of the biological tissue in the three-dimensional data.

In one embodiment, the control unit is configured to analyze the tomographic data to detect a position of the biological tissue with which the distal end of the catheter is in contact, and specify a point corresponding to the detected position as the contact point in the three-dimensional data.

In one embodiment, the control unit is configured to analyze the three-dimensional data to specify the contact point.

In one embodiment, the control unit is configured to receive input of position data indicating a position of the biological tissue with which the distal end of the catheter is in contact, and correct an analysis result of the three-dimensional data with reference to the position data.

In one embodiment, the control unit is configured to receive input of position data indicating a position of the biological tissue with which the distal end of the catheter is in contact, and specify a point corresponding to the position indicated by the position data as the contact point in the three-dimensional data.

In one embodiment, the control unit is configured to calculate a distance from the distal end of the catheter to each point of the biological tissue within a certain range in the three-dimensional data including the contact point, and adjust a ratio for including an element of the predetermined color in a color of each voxel of a voxel group of the three-dimensional image corresponding to the range in accordance with the calculated distance.

In one embodiment, when the control unit receives input of cauterization data indicating that a partial region of the biological tissue including the contact point is cauterized by an ablation catheter as the catheter, the control unit sets a color of a voxel group corresponding to the region to a color different from the predetermined color.

A diagnostic assistance system according to an aspect of the present disclosure includes the diagnostic assistance device and a sensor configured to acquire the tomographic data while moving in the biological tissue.

In one embodiment, the diagnostic assistance system further includes the display.

A diagnostic assistance method according to an aspect of the present disclosure is a diagnostic assistance method for generating three-dimensional data of a biological tissue inserted with a catheter based on tomographic data of the biological tissue, and displaying the generated three-dimensional data as a three-dimensional image on a display. The diagnostic assistance method includes: specifying a point of the biological tissue with which a distal end of the catheter is in contact as a contact point in the three-dimensional data; and setting a color of a voxel corresponding to the contact point to a predetermined color in the three-dimensional image.

Advantageous Effect

According to the present disclosure, it is easy to understand whether a catheter and a biological tissue are in contact with each other in a three-dimensional image.

Brief Description of Drawings

[FIG. 1] FIG. 1 is a perspective view of a diagnostic assistance system according to an embodiment.
[FIG. 2] FIG. 2 is a perspective view of a probe and a drive unit according to the embodiment.
[FIG. 3] FIG. 3 is a block diagram showing a configuration of a diagnostic assistance device according to the embodiment.
[FIG. 4] FIG. 4 is a flowchart showing an operation of the diagnostic assistance system according to the embodiment.
[FIG. 5] FIG. 5 is a diagram showing a positional relationship between a cross section of a biological tissue, an opening, and a viewpoint according to the embodiment.
[FIG. 6] FIG. 6 is a diagram showing a ratio of a size of a three-dimensional image to a screen of a display according to the embodiment, and coloring of a voxel group corresponding to a contact portion.
[FIG. 7] FIG. 7 is a flowchart showing processing performed in step S2 of FIG. 4.
[FIG. 8] FIG. 8 is a diagram showing processing performed in step S201 of FIG. 7.
[FIG. 9] FIG. 9 is a diagram showing processing performed in step S202 and step S203 of FIG. 7 in a modification.
[FIG. 10] FIG. 10 is a diagram showing coloring of a voxel group corresponding to a cauterization region according to a modification.
[FIG. 11] FIG. 11 is a flowchart showing processing performed in step S2 of FIG. 4 according to a modification.

Description of Embodiments

Hereinafter, embodiments will be described with reference to the drawings.

In the drawings, the same or corresponding parts are denoted by the same reference numerals. In the description of the present embodiment, the description of the same or corresponding parts will be omitted or simplified as appropriate.

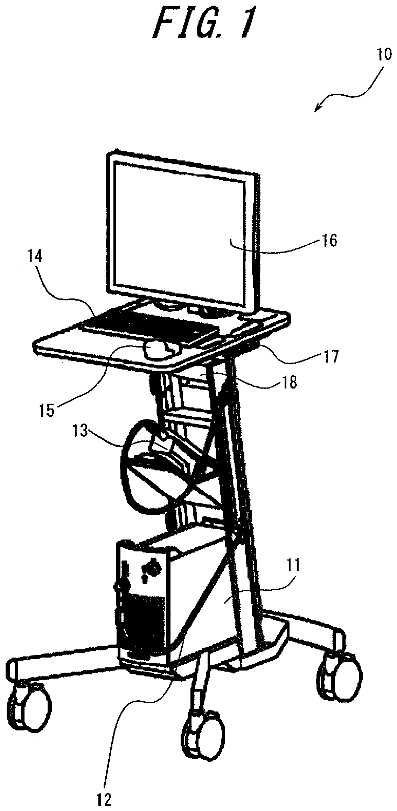

An outline of the present embodiment will be described with reference to FIGS. 1, 3, and 5.

A diagnostic assistance device 11 according to the present embodiment generates three-dimensional data 52 of a biological tissue 60 inserted with a catheter 63 based on tomographic data 51 of the biological tissue 60. The diagnostic assistance device 11 displays the generated three-dimensional data 52 as a three-dimensional image 53 on a display 16. The diagnostic assistance device 11 specifies a point of the biological tissue 60 with which a distal end of the catheter 63 is in contact as a contact point Pi in the three-dimensional data 52. The diagnostic assistance device 11 sets a color of a voxel corresponding to the contact point Pi to a predetermined color in the three-dimensional image 53.

The present embodiment facilitates understanding of whether the catheter 63 and the biological tissue 60 are in contact with each other in the three-dimensional image 53. For example, when a user is an operator who performs an ablation procedure, it is easy to understand whether an ablation catheter and a tissue in a cardiac cavity are in contact with each other, and thus it is easy to perform the ablation procedure.

The biological tissue 60 is, for example, an organ such as a blood vessel or a heart.

A configuration of a diagnostic assistance system 10 according to the present embodiment will be described with reference to FIG. 1.

The diagnostic assistance system 10 includes the diagnostic assistance device 11, a cable 12, a drive unit 13, a keyboard 14, a mouse 15, and the display 16.

The diagnostic assistance device 11 is a dedicated computer specialized for image diagnosing in the present embodiment, but may also be a general-purpose computer such as a PC. The term "PC" is an abbreviation of personal computer.

The cable 12 is used to connect the diagnostic assistance device 11 and the drive unit 13.

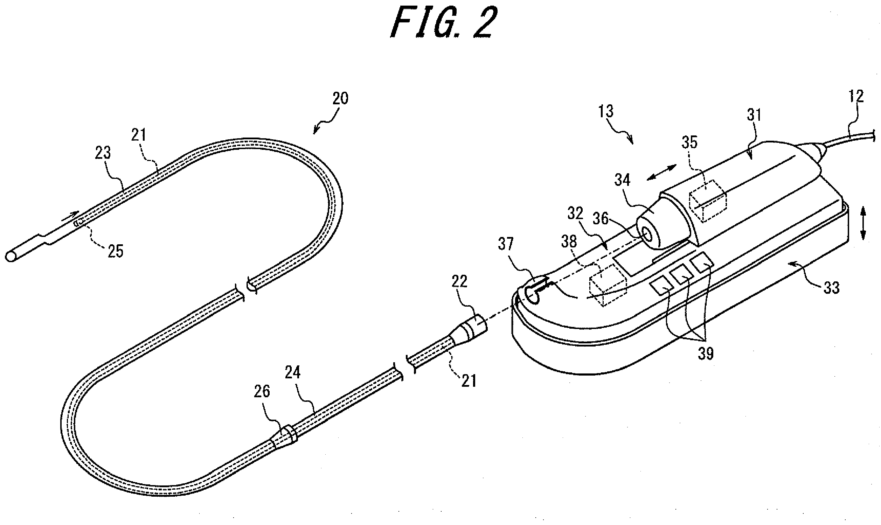

The drive unit 13 is a device to be used by connecting to a probe 20 shown in FIG. 2 to drive the probe 20. The drive unit 13 is also referred to as an MDU. The term "MDU" is an abbreviation of motor drive unit. The probe 20 is applied to IVUS. The probe 20 is also referred to as an IVUS catheter or an image diagnostic catheter.

The keyboard 14, the mouse 15, and the display 16 are connected to the diagnostic assistance device 11 via any cable or wirelessly. The display 16 is, for example, an LCD, an organic EL display, or an HMD. The term "LCD" is an abbreviation of liquid crystal display. The term "EL" is an abbreviation of electro luminescence. The term "HMD" is an abbreviation of head-mounted display.

The diagnostic assistance system 10 optionally further includes a connection terminal 17 and a cart unit 18.

The connection terminal 17 is used to connect the diagnostic assistance device 11 and an external device. The connection terminal 17 is, for example, a USB terminal. The term "USB" is an abbreviation of universal serial bus. The external device is, for example, a recording medium such as a magnetic disc drive, a magneto-optical disc drive, or an optical disc drive.

The cart unit 18 is a cart equipped with casters for movement. The diagnostic assistance device 11, the cable 12, and the drive unit 13 are disposed on a cart body of the cart unit 18. The keyboard 14, the mouse 15, and the display 16 are disposed on an uppermost table of the cart unit 18.

A configuration of the probe 20 and the drive unit 13 according to the present embodiment will be described with reference to FIG. 2.

The probe 20 includes a drive shaft 21, a hub 22, a sheath 23, an outer tube 24, an ultrasound transducer 25, and a relay connector 26.

The drive shaft 21 passes through the sheath 23 to be inserted into a body cavity of a living body and the outer tube 24 connected to a proximal end of the sheath 23, and extends to an inside of the hub 22 provided at a proximal end of the probe 20. The drive shaft 21 is provided with the ultrasound transducer 25, which transmits and receives signals, at a distal end thereof, and is rotatably provided in the sheath 23 and the outer tube 24. The relay connector 26 connects the sheath 23 and the outer tube 24.

The hub 22, the drive shaft 21, and the ultrasound transducer 25 are connected to each other so as to integrally move forward and backward in an axial direction. Therefore, for example, when the hub 22 is pressed toward a distal side, the drive shaft 21 and the ultrasound transducer 25 move inside the sheath 23 toward the distal side. For example, when the hub 22 is pulled toward a proximal side, the drive shaft 21 and the ultrasound transducer 25 move inside the sheath 23 toward the proximal side as indicated by arrows.

The drive unit 13 includes a scanner unit 31, a slide unit 32, and a bottom cover 33.

The scanner unit 31 is connected to the diagnostic assistance device 11 via the cable 12. The scanner unit 31 includes a probe connection unit 34 connected to the probe 20, and a scanner motor 35 which is a drive source for rotating the drive shaft 21.

The probe connection unit 34 is detachably connected to the probe 20 through an insertion port 36 of the hub 22 provided at the proximal end of the probe 20. Inside the hub 22, a proximal end of the drive shaft 21 is rotatably supported, and a rotational force of the scanner motor 35 is transmitted to the drive shaft 21. A signal is transmitted and received between the drive shaft 21 and the diagnostic assistance device 11 via the cable 12. In the diagnostic assistance device 11, a tomographic image of a living body lumen is generated and image processing is performed based on the signal transmitted from the drive shaft 21.

The slide unit 32 is mounted with the scanner unit 31 in a manner capable of moving forward and backward, and is mechanically and electrically connected to the scanner unit 31. The slide unit 32 includes a probe clamp unit 37, a slide motor 38, and a switch group 39.

The probe clamp unit 37 is disposed coaxially with the probe connection unit 34 on a distal side of the probe connection unit 34, and supports the probe 20 to be connected to the probe connection unit 34.

The slide motor 38 is a drive source that generates a driving force in the axial direction. The scanner unit 31 moves forward and backward when driven by the slide motor 38, and the drive shaft 21 moves forward and backward in the axial direction accordingly. The slide motor 38 is, for example, a servo motor.

The switch group 39 includes, for example, a forward switch and a pull-back switch that are pressed when the scanner unit 31 is to be moved forward or backward, and a scan switch that is pressed when image drawing is to be started or ended. Various switches may be included in the switch group 39 as necessary without being limited to the example here.

When the forward switch is pressed, the slide motor 38 rotates forward, and the scanner unit 31 moves forward. On the other hand, when the pull-back switch is pressed, the slide motor 38 rotates backward, and the scanner unit 31 moves backward.

When the scan switch is pressed, the image drawing is started, the scanner motor 35 is driven, and the slide motor 38 is driven to move the scanner unit 31 backward. A user such as an operator connects the probe 20 to the scanner unit 31 in advance, and rotates and moves the drive shaft 21 toward the proximal side in the axial direction upon the start of the image drawing. When the scan switch is pressed again, the scanner motor 35 and the slide motor 38 are stopped, and the image drawing is ended.

The bottom cover 33 covers a bottom and an entire circumference of a side surface on a bottom side of the slide unit 32, and is capable of moving toward and away from the bottom of the slide unit 32.

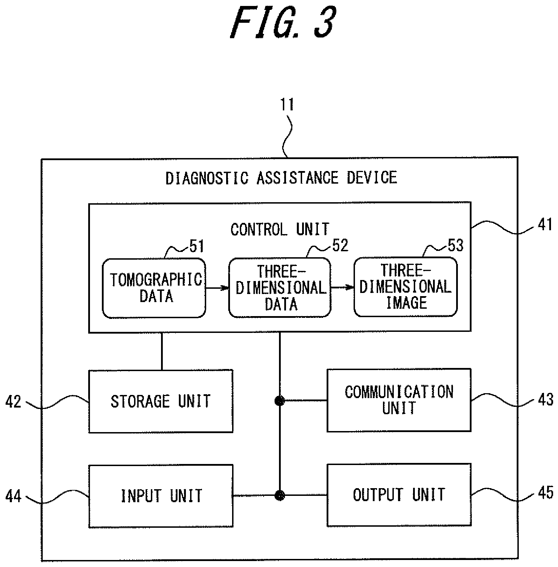

A configuration of the diagnostic assistance device 11 according to the present embodiment will be described with reference to FIG. 3.

The diagnostic assistance device 11 includes a control unit 41, a storage unit 42, a communication unit 43, an input unit 44, and an output unit 45.

The control unit 41 includes at least one processor, at least one dedicated circuit, or a combination thereof. The processor is a general-purpose processor such as a CPU or GPU, or a dedicated processor specialized for a specific process. The term "CPU" is an abbreviation of central processing unit. The term "GPU" is an abbreviation of graphics processing unit. As the dedicated circuit, for example, an FPGA or an ASIC can be used. The term "FPGA" is an abbreviation of field-programmable gate array. The term "ASIC" is an abbreviation of application specific integrated circuit. The control unit 41 executes processing related to an operation of the diagnostic assistance device 11 while controlling each unit of the diagnostic assistance system 10 including the diagnostic assistance device 11.

The storage unit 42 includes at least one semiconductor memory, at least one magnetic memory, at least one optical memory, or a combination of at least two thereof. The semiconductor memory is, for example, a RAM or a ROM. The term "RAM" is an abbreviation of random access memory. The term "ROM" is an abbreviation of read only memory. The RAM is, for example, an SRAM or a DRAM. The term "SRAM" is an abbreviation of static random access memory. The term "DRAM" is an abbreviation of dynamic random access memory. The ROM is, for example, an EEPROM. The term "EEPROM" is an abbreviation of electrically erasable programmable read only memory. The storage unit 42 functions as, for example, a main storage device, an auxiliary storage device, or a cache memory. The storage unit 42 stores data used for the operation of the diagnostic assistance device 11, such as the tomographic data 51, and data obtained by the operation of the diagnostic assistance device 11, such as the three-dimensional data 52 and the three-dimensional image 53.

The communication unit 43 includes at least one communication interface. The communication interface is a wired LAN interface, a wireless LAN interface, or an image diagnostic interface for receiving IVUS signals and performing A/D conversion on the IVUS signals. The term "LAN" is an abbreviation of local area network. The term "A/D" is an abbreviation of analog to digital. The communication unit 43 receives data used for the operation of the diagnostic assistance device 11 and transmits data obtained by the operation of the diagnostic assistance device 11. In the present embodiment, the drive unit 13 is connected to the image diagnostic interface included in the communication unit 43.

The input unit 44 includes at least one input interface. The input interface is, for example, a USB interface, an HDMI (registered trademark) interface, or an interface compatible with short-range wireless communication such as Bluetooth (registered trademark). The term "HDMI (registered trademark)" is an abbreviation of high-definition multimedia interface. The input unit 44 receives an operation of inputting data used for the operation of the diagnostic assistance device 11. In the present embodiment, the keyboard 14 and the mouse 15 are connected to the USB interface or the interface corresponding to short-range wireless communication included in the input unit 44. When a touch screen is provided integrally with the display 16, the display 16 may be connected to the USB interface or the HDMI (registered trademark) interface included in the input unit 44.

The output unit 45 includes at least one output interface. The output interface is, for example, a USB interface, an HDMI (registered trademark) interface, or an interface compatible with short-range wireless communication such as Bluetooth (registered trademark). The output unit 45 outputs the data obtained by the operation of the diagnostic assistance device 11. In the present embodiment, the display 16 is connected to the USB interface or the HDMI (registered trademark) interface included in the output unit 45.

A function of the diagnostic assistance device 11 is implemented by executing a diagnostic assistance program according to the present embodiment by the processor included in the control unit 41. That is, the function of the diagnostic assistance device 11 is implemented by software. The diagnostic assistance program is a program for causing a computer to execute the processing of steps included in the operation of the diagnostic assistance device 11 to implement a function corresponding to the processing of the steps. That is, the diagnostic assistance program is a program for causing the computer to function as the diagnostic assistance device 11.

The program can be recorded in a computer-readable recording medium. The computer-readable recording medium is, for example, a magnetic recording device, an optical disk, a magneto-optical recording medium, or a semiconductor memory. The program is distributed by, for example, selling, transferring, or lending a portable recording medium such as a DVD or a CD-ROM on which the program is recorded. The term "DVD" is an abbreviation of digital versatile disc. The term "CD-ROM" is an abbreviation of compact read only memory. The program may be distributed by storing the program in a storage of a server and transferring the program from the server to another computer via a network. The program may be provided as a program product.

For example, the computer temporarily stores the program recorded in the portable recording medium or the program transferred from the server in the main storage device. The computer causes the processor to read the program stored in the main storage device, and causes the processor to execute processing according to the read program. The computer may read the program directly from the portable recording medium and execute the processing according to the program. Each time the program is transferred from the server to the computer, the computer may sequentially execute processing according to the received program. The processing may be executed by a so-called ASP type service in which the function is implemented only by execution instruction and result acquisition without transferring the program from the server to the computer. The term "ASP" is an abbreviation of application service provider. The program includes information provided for processing by an electronic computer and conforming to the program. For example, data that is not a direct command to the computer but has a property that defines the processing of the computer corresponds to the "information conforming to the program".

The functions of the diagnostic assistance device 11 may be partially or entirely implemented by the dedicated circuit included in the control unit 41. That is, the functions of the diagnostic assistance device 11 may be partially or entirely implemented by hardware.

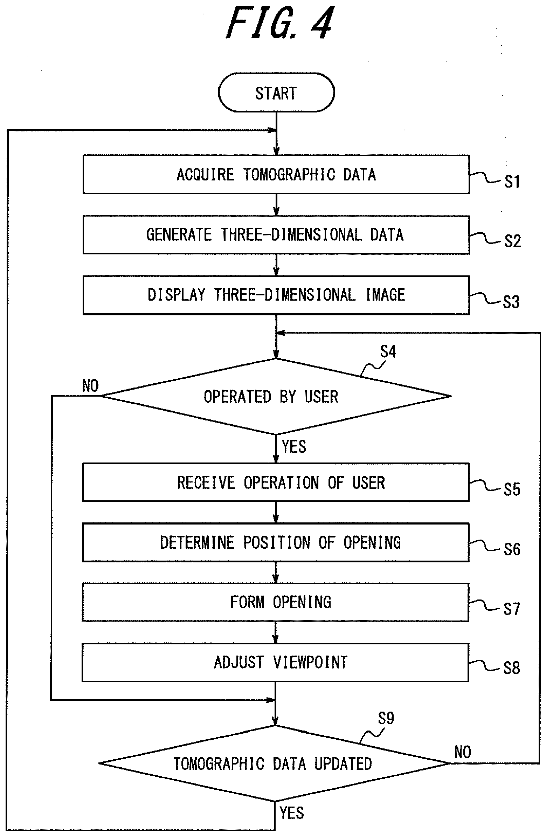

An operation of the diagnostic assistance system 10 according to the present embodiment will be described with reference to FIG. 4. The operation of the diagnostic assistance system 10 corresponds to a diagnostic assistance method according to the present embodiment.

Before the start of a flow in FIG. 4, the probe 20 is primed by the user. Thereafter, the probe 20 is fitted into the probe connection unit 34 and the probe clamp unit 37 of the drive unit 13, and is connected and fixed to the drive unit 13. The probe 20 is inserted to a target site in the biological tissue 60 such as the blood vessel or the heart.

In step S1, the scan switch included in the switch group 39 is pressed, and a so-called pull-back operation is performed by pressing the pull-back switch included in the switch group 39. The probe 20 transmits ultrasound inside the biological tissue 60 by the ultrasound transducer 25 that moves backward in the axial direction by the pullback operation. The ultrasound transducer 25 radially transmits the ultrasound while moving inside the biological tissue 60. The ultrasonic transducer 25 receives a reflected wave of the transmitted ultrasound. The probe 20 inputs a signal of the reflected wave received by the ultrasound transducer 25 to the diagnostic assistance device 11. The control unit 41 of the diagnostic assistance device 11 processes the input signal to sequentially generate cross-sectional images of the biological tissue 60, thereby acquiring the tomographic data 51, which includes a plurality of cross-sectional images.

Specifically, the probe 20 transmits the ultrasound in a plurality of directions from a rotation center to an outside by the ultrasound transducer 25 while causing the ultrasound transducer 25 to rotate in a circumferential direction and to move in the axial direction inside the biological tissue 60. The probe 20 receives the reflected wave from a reflecting object existing in each of the plurality of directions inside the biological tissue 60 by the ultrasound transducer 25. The probe 20 transmits the signal of the received reflected wave to the diagnostic assistance device 11 via the drive unit 13 and the cable 12. The communication unit 43 of the diagnostic assistance device 11 receives the signal transmitted from the probe 20. The communication unit 43 performs A/D conversion on the received signal. The communication unit 43 inputs the A/D-converted signal to the control unit 41. The control unit 41 processes the input signal to calculate an intensity value distribution of the reflected wave from the reflecting object existing in a transmission direction of the ultrasound of the ultrasound transducer 25. The control unit 41 sequentially generates two-dimensional images having a luminance value distribution corresponding to the calculated intensity value distribution as the cross-sectional images of the biological tissue 60, thereby acquiring the tomographic data 51 which is a data set of the cross-sectional images. The control unit 41 stores the acquired tomographic data 51 in the storage unit 42.

In the present embodiment, the signal of the reflected wave received by the ultrasound transducer 25 corresponds to raw data of the tomographic data 51, and the cross-sectional images generated by processing the signal of the reflected wave by the diagnostic assistance device 11 correspond to processed data of the tomographic data 51.

In a modification of the present embodiment, the control unit 41 of the diagnostic assistance device 11 may store the signal input from the probe 20 as it is in the storage unit 42 as the tomographic data 51. Alternatively, the control unit 41 may store data indicating the intensity value distribution of the reflected wave calculated by processing the signal input from the probe 20 in the storage unit 42 as the tomographic data 51. That is, the tomographic data 51 is not limited to the data set of the cross-sectional images of the biological tissue 60, and may be data representing a cross section of the biological tissue 60 at each moving position of the ultrasound transducer 25 in some format.

In a modification of the present embodiment, an ultrasound transducer that transmits ultrasound in a plurality of directions without rotating may be used instead of the ultrasound transducer 25 that transmits ultrasound in the plurality of directions while rotating in the circumferential direction.

In a modification of the present embodiment, the tomographic data 51 may be acquired using OFDI or OCT instead of being acquired by using the IVUS. The term "OFDI" is an abbreviation of optical frequency domain imaging. The term "OCT" is an abbreviation of optical coherence tomography. When OFDI or OCT is used, as a sensor that acquires the tomographic data 51 while moving in the biological tissue 60, a sensor that acquires the tomographic data 51 by emitting light in the biological tissue 60 is used instead of the ultrasound transducer 25 that acquires the tomographic data 51 by transmitting the ultrasound in the biological tissue 60.

In a modification of the present embodiment, instead of the diagnostic assistance device 11 generating the data set of the cross-sectional images of the biological tissue 60, another device may generate the same data set, and the diagnostic assistance device 11 may acquire the data set from the other device. That is, instead of the control unit 41 of the diagnostic assistance device 11 processing the IVUS signal to generate the cross-sectional image of the biological tissue 60, another device may process the IVUS signal to generate the cross-sectional image of the biological tissue 60 and input the generated cross-sectional image to the diagnostic assistance device 11.

In step S2, the control unit 41 of the diagnostic assistance device 11 generates the three-dimensional data 52 of the biological tissue 60 based on the tomographic data 51 acquired in step S1.

Specifically, the control unit 41 of the diagnostic assistance device 11 generates the three-dimensional data 52 of the biological tissue 60 by stacking the cross-sectional images of the biological tissue 60 included in the tomographic data 51 stored in the storage unit 42, and converting the same into three-dimensional data. As a method of three-dimensional conversion, any process among a rendering method such as surface rendering or volume rendering, texture mapping accompanying the rendering method, such as environment mapping and bump mapping, or the like is used. The control unit 41 stores the generated three-dimensional data 52 in the storage unit 42.

When the catheter 63 other than the IVUS catheter, such as an ablation catheter, is inserted into the biological tissue 60, the tomographic data 51 includes data of the catheter 63 as well as data of the biological tissue 60. Therefore, in step S2, the three-dimensional data 52 generated by the control unit 41 also includes data of the catheter 63 as well as data of the biological tissue 60.

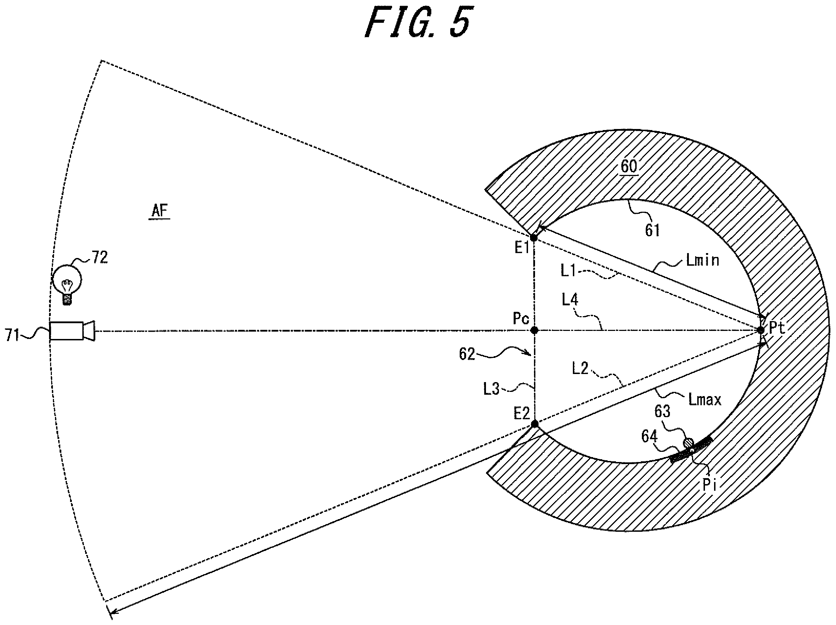

As shown in FIG. 5, the control unit 41 specifies the point of the biological tissue 60 with which the distal end of the catheter 63 is in contact as the contact point Pi in the three-dimensional data 52. The control unit 41 sets the color of the voxel corresponding to the contact point Pi to the predetermined color in the three-dimensional image 53 displayed in step S3. The term "predetermined color" is red in the present embodiment, but may also be any color as long as the voxel corresponding to the contact point Pi can be distinguished from other voxel groups.

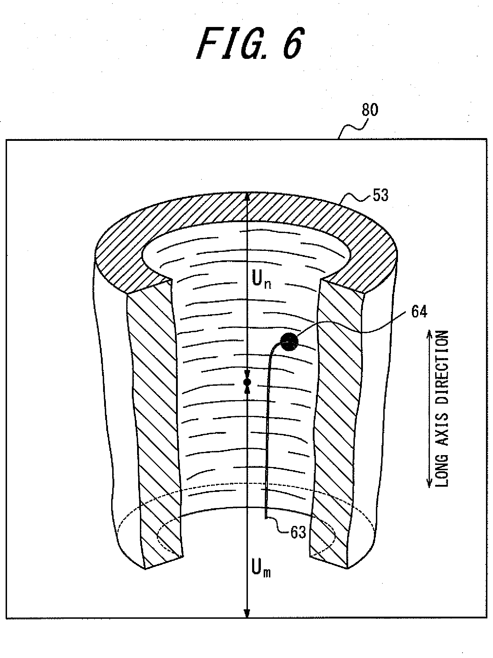

In the present embodiment, as shown in FIGS. 5 and 6, the control unit 41 specifies a certain range of the biological tissue 60 including the contact point Pi in the three-dimensional data 52 as a contact portion 64. The control unit 41 sets a color of the voxel group corresponding to the contact portion 64 to a predetermined color in the three-dimensional image 53 displayed in step S3.

Specifically, the control unit 41 analyzes the tomographic data 51 stored in the storage unit 42 to detect a position of the biological tissue 60 with which the distal end of the catheter 63 is in contact. Any method may be used for analyzing the tomographic data 51, and the present embodiment uses a method of determining whether the biological tissue 60 and the distal end of the catheter 63 are in contact with each other by detecting the biological tissue 60 and the catheter 63 in the cross-sectional images included in the tomographic data 51 and measuring a distance between the biological tissue 60 and the distal end of the catheter 63. The control unit 41 specifies a point corresponding to the detected position as the contact point Pi in the three-dimensional data 52.

In a modification of the present embodiment, the control unit 41 may analyze the three-dimensional data 52 to specify the contact point Pi. Any method may be used as a method of analyzing the three-dimensional data 52, and for example, a method of determining whether the biological tissue 60 and the distal end of the catheter 63 are in contact with each other by detecting the distal end of the catheter 63 included in the three-dimensional data 52 and measuring the distance between the biological tissue 60 and the distal end of the catheter 63 is used.

In the modification, the control unit 41 may receive input of position data indicating the position of the biological tissue 60 with which the distal end of the catheter 63 is in contact. Specifically, the control unit 41 may receive input of the position data via the communication unit 43 or the input unit 44 from an external system that determines whether the distal end of the catheter 63 is in contact with an inner wall of the biological tissue 60 using a sensor such as an electrode provided at the distal end of the catheter 63. The control unit 41 may correct an analysis result of the three-dimensional data 52 with reference to the input position data.

In a modification of the present embodiment, the control unit 41 may specify a point corresponding to a position indicated by the position data input from the external system as described above as the contact point Pi in the three-dimensional data 52 without analyzing the three-dimensional data 52.

In step S3, the control unit 41 of the diagnostic assistance device 11 displays the three-dimensional data 52 generated in step S2 on the display 16 as the three-dimensional image 53. At this time, the control unit 41 may arrange a viewpoint and virtual light sources 72 at any position when the three-dimensional image 53 is to be displayed on the display 16. The term "viewpoint" refers to a position of a virtual camera 71 as shown in FIG. 5 to be arranged in a three-dimensional space. The number and the relative positions of the light sources 72 are not limited to those illustrated in the drawings, and can be changed as appropriate.

Specifically, the control unit 41 of the diagnostic assistance device 11 generates the three-dimensional image 53 from the three-dimensional data 52 stored in the storage unit 42. The control unit 41 displays the generated three-dimensional image 53 on the display 16 via the output unit 45.

In step S4, when operated by the user, the processing from step S5 to step S8 is performed. When not operated by the user, the processing from step S5 to step S8 is skipped.

In step S5, the control unit 41 of the diagnostic assistance device 11 receives, via the input unit 44, an operation of setting a position of an opening 62 as shown in FIG. 5. The position of the opening 62 is set to a position at which an inner wall surface 61 of the biological tissue 60 is exposed to the outside of the biological tissue 60 through the opening 62 in the three-dimensional image 53 displayed in step S3.

Specifically, the control unit 41 of the diagnostic assistance device 11 receives, via the input unit 44, an operation of the user cutting off a portion of the biological tissue 60 using the keyboard 14, the mouse 15, or a touch screen provided integrally with the display 16 in the three-dimensional image 53 displayed on the display 16. In the example of FIG. 5, the control unit 41 receives an operation of cutting off a portion of the biological tissue 60 so that the inner wall surface 61 of the biological tissue 60 has an opened shape in the cross section of the biological tissue 60. The term "cross section of the biological tissue 60" refers to, for example, a tomographic cross section having two end edges of the opening 62 facing each other and the inner wall surface 61 of the biological tissue 60 facing the opening 62, but is not limited to this tomographic cross section, and may be a transverse cross section of the biological tissue 60, a longitudinal cross section of the biological tissue 60, or another cross section of the biological tissue 60. The term "transverse cross section of the biological tissue 60" refers to a cross section obtained by cutting the biological tissue 60 perpendicularly to a direction in which the ultrasound transducer 25 moves in the biological tissue 60. The term "longitudinal cross section of the biological tissue 60" refers to a cross section obtained by cutting the biological tissue 60 along a direction in which the ultrasound transducer 25 moves in the biological tissue 60. The term "another cross section of the biological tissue 60" refers to a cross section obtained by cutting the biological tissue 60 obliquely with respect to a direction in which the ultrasound transducer 25 moves in the biological tissue 60. The term "opened shape" refers to, for example, a substantially C shape, a substantially U shape, a substantially "3" shape, or a shape in which any of these shapes is partially missing due to a hole originally opened in the biological tissue 60, such as a bifurcated portion of the blood vessel or pulmonary vein ostia. In the example of FIG. 5, a shape of the inner wall surface 61 of the biological tissue 60 is a substantially C shape.

In step S6, the control unit 41 of the diagnostic assistance device 11 determines the position set by the operation received in step S5 as the position of the opening 62.

Specifically, the control unit 41 of the diagnostic assistance device 11 specifies, as three-dimensional coordinates of an edge of the opening 62, three-dimensional coordinates of a boundary of a portion of the biological tissue 60 cut off by the operation of the user in the three-dimensional data 52 stored in the storage unit 42. The control unit 41 stores the specified three-dimensional coordinates in the storage unit 42.

In step S7, the control unit 41 of the diagnostic assistance device 11 forms, in the three-dimensional data 52, the opening 62 that exposes the inner wall surface 61 of the biological tissue 60 to the outside of the biological tissue 60 in the three-dimensional image 53.

Specifically, the control unit 41 of the diagnostic assistance device 11 sets a portion in the three-dimensional data 52 stored in the storage unit 42 that is specified by the three-dimensional coordinates stored in the storage unit 42 to be hidden or transparent when the three-dimensional image 53 is to be displayed on the display 16.

In step S8, the control unit 41 of the diagnostic assistance device 11 adjusts the viewpoint when displaying the three-dimensional image 53 on the display 16 according to the position of the opening 62 formed in step S7. In the present embodiment, the control unit 41 arranges the viewpoint on a straight line extending from the inner wall surface 61 of the biological tissue 60 to the outside of the biological tissue 60 through the opening 62. Therefore, the user can virtually observe the inner wall surface 61 of the biological tissue 60 by looking into the biological tissue 60 through the opening 62.

Specifically, the control unit 41 of the diagnostic assistance device 11 arranges the virtual camera 71 at a position where the inner wall surface 61 of the biological tissue 60 can be seen through the portion set to be hidden or transparent in the three-dimensional image 53 displayed on the display 16. In the example of FIG. 5, the control unit 41 arranges the virtual camera 71 in a region AF sandwiched between a first straight line L1 and a second straight line L2 in the cross section of the biological tissue 60. The first straight line L1 extends from the inner wall surface 61 of the biological tissue 60 to the outside of the biological tissue 60 through a first end edge E1 of the opening 62. The second straight line L2 extends from the inner wall surface 61 of the biological tissue 60 to the outside of the biological tissue 60 through a second end edge E2 of the opening 62. A point at which the first straight line L1 intersects the inner wall surface 61 of the biological tissue 60 is a point Pt identical to a point at which the second straight line L2 intersects the inner wall surface 61 of the biological tissue 60. Therefore, the user can observe the point Pt on the inner wall surface 61 of the biological tissue 60 regardless of a position of the virtual camera 71 in the region AF.

In the example of FIG. 5, the point Pt is identical to a point at which a fourth straight line L4 intersects the inner wall surface 61 of the biological tissue 60. The fourth straight line L4 is drawn perpendicularly to a third straight line L3 from a midpoint Pc of the third straight line L3. The third straight line L3 connects the first end edge E1 of the opening 62 and the second end edge E2 of the opening 62. Therefore, the user can easily observe the point Pt on the inner wall surface 61 of the biological tissue 60 through the opening 62. In particular, as shown in FIG. 5, when the virtual camera 71 is arranged on an extension line of the fourth straight line L4, the user can easily observe the point Pt on the inner wall surface 61 of the biological tissue 60.

The position of the virtual camera 71 may be any position at which the inner wall surface 61 of the biological tissue 60 can be observed through the opening 62, and is within a range facing the opening 62 in the present embodiment. The position of the virtual camera 71 is preferably set to an intermediate position facing a central portion of the opening 62.

In the example of FIG. 6, a minimum value Smin and a maximum value Smax are set for a ratio S of a distance Un from a center to one end of the three-dimensional image 53 displayed on a screen 80 of the display 16 to a distance Um from a center to one end of the screen 80 such that the centers of the screen 80 and the three-dimensional image 53 overlap with each other. For example, Smin is set to 1/3, and Smax is set to 1. In the example of FIG. 5, a minimum distance Lmin from the point Pt to the position of the camera 71 may be set according to the minimum value Smin, and a maximum distance Lmax from the point Pt to the position of the virtual camera 71 may be set according to the maximum value Smax. Alternatively, the minimum distance Lmin from the point Pt to the position of the camera 71 may be set to such a distance that the camera 71 is not closer to the point Pt than the opening 62 regardless of the minimum value Smin. The maximum distance Lmax from the point Pt to the position of the virtual camera 71 may be set to such a distance that the camera 71 is not away from the point Pt more than such a distant that the user cannot observe the inner wall surface 61 of the biological tissue 60 regardless of the maximum value Smax.

In step S9, when the tomographic data 51 is updated, the processing of step S1 and the subsequent steps are performed again. When the tomographic data 51 is not updated, whether operated by the user is confirmed again in step S4.

In steps S5 to S8 for the second and subsequent times, when the position of the opening 62 is changed from a first position to a second position, the control unit 41 of the diagnostic assistance device 11 moves the viewpoint from a third position corresponding to the first position to a fourth position corresponding to the second position. The control unit 41 moves the virtual light sources 72 when the three-dimensional image 53 is to be displayed on the display 16, in accordance with movement of the viewpoint from the third position to the fourth position.

The control unit 41 moves the virtual light sources 72 by using a rotation matrix used for moving the virtual camera 71 when changing a position of the opening 62 in the circumferential direction in the cross section of the biological tissue 60.

The control unit 41 may instantly switch the viewpoint from the third position to the fourth position when changing the position of the opening 62 from the first position to the second position, but in the present embodiment, a video in which the viewpoint gradually moves from the third position to the fourth position is displayed on the display 16 as the three-dimensional image 53. Therefore, the movement of the viewpoint is easily introduced to the user.

In a modification of the present embodiment, in step S5, the control unit 41 of the diagnostic assistance device 11 may receive, via the input unit 44, an operation of setting a position of a target point that the user wants to see and an operation of setting the position of the opening 62.

Specifically, in the three-dimensional image 53 displayed on the display 16, the control unit 41 of the diagnostic assistance device 11 may receive, via the input unit 44, an operation of designating the position of the target point using the keyboard 14, the mouse 15, or the touch screen provided integrally with the display 16 by the user. In the example of FIG. 5, the control unit 41 may receive, via the input unit 44, an operation of setting a position of the point Pt as a position of the point at which the first straight line L1 and the second straight line L2 intersect the inner wall surface 61 of the biological tissue 60.

In a modification of the present embodiment, in step S5, the control unit 41 of the diagnostic assistance device 11 may receive, via the input unit 44, an operation of setting the position of the target point that the user wants to see, instead of the operation of setting the position of the opening 62. In step S6, the control unit 41 may determine the position of the opening 62 according to the position set by the operation received in step S5.

Specifically, in the three-dimensional image 53 displayed on the display 16, the control unit 41 of the diagnostic assistance device 11 may receive, via the input unit 44, the operation of designating the position of the target point using the keyboard 14, the mouse 15, or the touch screen provided integrally with the display 16 by the user. The control unit 41 may determine the position of the opening 62 according to the position of the target point. In the example of FIG. 5, the control unit 41 may receive, via the input unit 44, an operation of setting the position of the point Pt as the position of the point at which the first straight line L1 and the second straight line L2 intersect the inner wall surface 61 of the biological tissue 60. In the cross section of the biological tissue 60, the control unit 41 may determine, as the region AF, a fan-shaped region centered on the point Pt and having a central angle that is preset or that is an angle $\alpha$ specified by the user. The control unit 41 may determine a position in the biological tissue 60 that overlaps with the region AF as the position of the opening 62. The control unit 41 may determine a normal line of the inner wall surface 61 of the biological tissue 60 perpendicular to a tangent line passing through the point Pt as the fourth straight line L4.

The region AF may be set to be narrower than a width of the opening 62. That is, the region AF may be set so as not to include at least one of the first end edge E1 of the opening 62 and the second end edge E2 of the opening 62.

In a modification of the present embodiment, the point at which the first straight line L1 intersects the inner wall surface 61 of the biological tissue 60 may not be identical to the point at which the second straight line L2 intersects the inner wall surface 61 of the biological tissue 60. For example, a point P1 at which the first straight line L1 intersects the inner wall surface 61 of the biological tissue 60 and a point P2 at which the second straight line L2 intersects the inner wall surface 61 of the biological tissue 60 may be on a circumference centered on the point Pt. That is, the point P1 and the point P2 may be substantially equidistant from the point Pt.

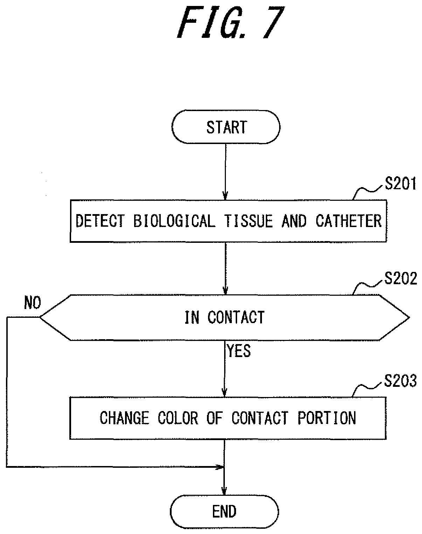

The details of the processing performed in step S2 when the catheter 63 is inserted into the biological tissue 60 will be described with reference to FIG. 7.

In step S201, the control unit 41 of the diagnostic assistance device 11 detects the biological tissue 60 and the catheter 63 in the cross-sectional image included in the tomographic data 51 acquired in step S1.

Specifically, the control unit 41 of the diagnostic assistance device 11 classifies a pixel group of the cross-sectional image included in the tomographic data 51 acquired in step S1 into two or more classes. The two or more classes include at least a class of the biological tissue 60 and a class of the catheter 63, and may further include a class of blood cell, a class of medical instrument other than the catheter 63 such as a guide wire, a class of indwelling such as a stent, and a class of lesion such as lime or plaque. Any method may be used as a classification method, and the present embodiment uses a method of classifying the pixel group of the cross-sectional image by a learned model. The learned model is trained such that a region corresponding to each class can be detected from a cross-sectional image of IVUS as a sample by performing machine learning in advance.

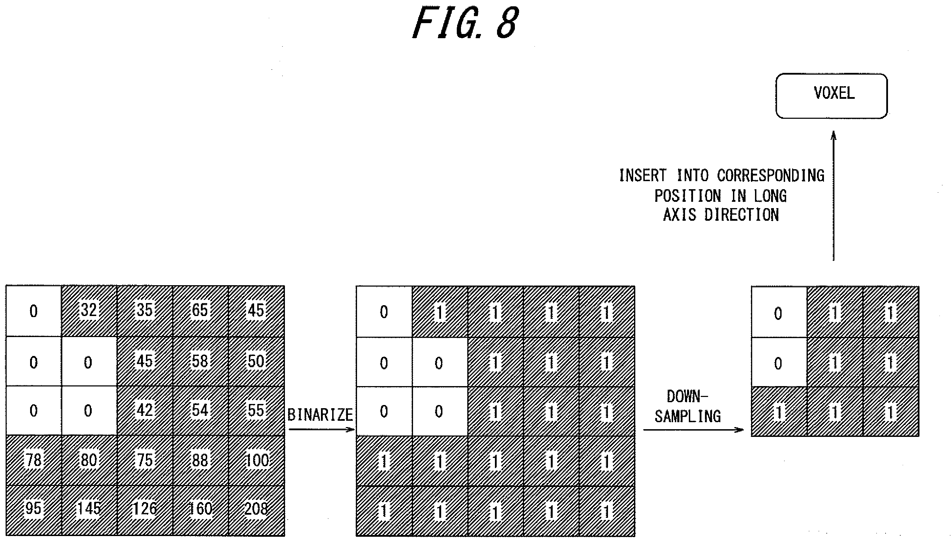

In the example of FIG. 8, the control unit 41 binarizes each pixel of the cross-sectional image included in the tomographic data 51 so that a pixel value becomes 1 when the pixel value corresponds to the class of the biological tissue 60, and otherwise becomes 0. The control unit 41 performs down-sampling of the binarized cross-sectional image, and sets each pixel of the obtained two-dimensional image as a voxel at a position corresponding to the two-dimensional image in a long axis direction of the biological tissue 60.

In a modification of the present embodiment, the control unit 41 may detect the biological tissue 60 by extracting an edge in the cross-sectional image.

In step S202, the control unit 41 of the diagnostic assistance device 11 determines whether the biological tissue 60 and the distal end of the catheter 63 are in contact with each other by measuring the distance between the biological tissue 60 and the distal end of the catheter 63 detected in step S201.

Specifically, the control unit 41 of the diagnostic assistance device 11 lists the catheters 63 detected in step S201 to create a catheter list. The control unit 41 detects the distal end of the catheter 63 for each entry of the catheter list. When only one catheter 63 is detected in step S201, the number of entries in the catheter list is one. The control unit 41 lists the detected distal ends of the catheters 63 to create a catheter distal end list. The control unit 41 specifies a position on the inner wall surface 61 of the biological tissue 60 that is closest to the distal end of the catheter 63 for each entry of the catheter distal end list. The control unit 41 calculates a distance between the distal end of the catheter 63 and the specified position. When the calculated distance is less than a first threshold value, the control unit 41 determines that the biological tissue 60 and the distal end of the catheter 63 are in contact with each other. When the calculated distance is equal to or greater than the first threshold value, the control unit 41 determines that the biological tissue 60 and the distal end of the catheter 63 are not in contact with each other.

When it is determined in step S202 that the biological tissue 60 and the distal end of the catheter 63 are in contact with each other, in step S203, the control unit 41 of the diagnostic assistance device 11 specifies a range of the biological tissue 60 in which the distance between the biological tissue 60 and the distal end of the catheter 63 detected in step S201 is less than a certain value as the contact portion 64. The control unit 41 sets the color of the voxel group corresponding to the contact portion 64 to the predetermined color in the three-dimensional image 53 displayed in step S3.

Specifically, the control unit 41 of the diagnostic assistance device 11 specifies a range on the inner wall surface 61 of the biological tissue 60 in which a distance to the distal end of the catheter 63 is less than a second threshold value as the contact portion 64 for each entry of the catheter distal end list created in step S202. The control unit 41 sets the color of the voxel group corresponding to the contact portion 64 to red in the three-dimensional image 53 displayed in step S3.

When it is determined in step S202 that the biological tissue 60 and the distal end of the catheter 63 are not in contact with each other, the processing of step S203 is skipped.

In a modification of the present embodiment, the control unit 41 of the diagnostic assistance device 11 may adjust a ratio for including an element of the predetermined color in a color of each voxel of the three-dimensional image 53 in accordance with a distance from a distal end 63d of the catheter 63 to each point of the biological tissue 60 in the three-dimensional data 52. That is, in the three-dimensional image 53, the biological tissue 60 may be colored with gradation around a position where the catheter 63 and the biological tissue 60 are in contact with each other.

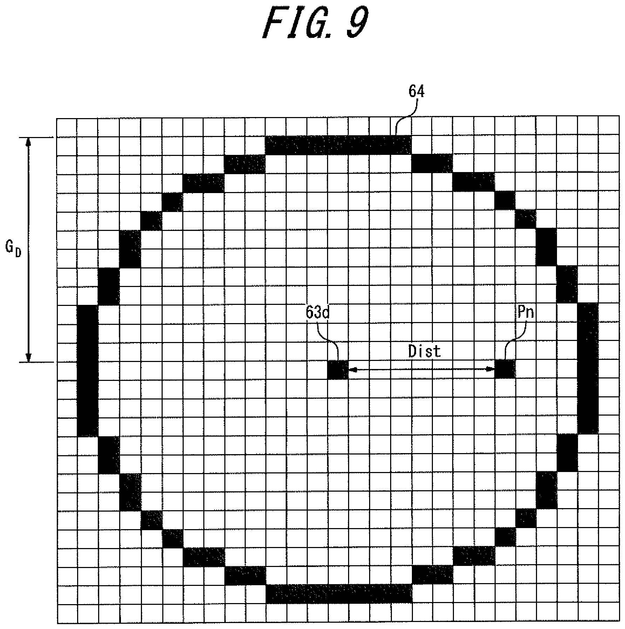

In the modification, as shown in FIG. 9, the control unit 41 calculates a distance Dist between the distal end 63d of the catheter 63 and each point Pn on the inner wall surface 61 of the biological tissue 60. FIG. 9 shows a two-dimensional grid for the sake of convenience, but processing is actually performed using a three-dimensional grid. When $G_D$ is a predetermined distance from the distal end 63d of the catheter 63 and $C_F$ is a contact coefficient, the control unit 41 calculates a contact coefficient of each voxel including the inner wall surface 61 of the biological tissue 60 by the following calculation.

$$C_F = 1 - \text{clamp}(\text{Dist}/G_D, 0, 1)$$

Here, clamp() is a clamp function. A voxel whose contact coefficient is close to 1 is closer to the distal end 63d of the catheter 63, and a voxel whose contact coefficient is close to 0 is farther from the distal end 63d of the catheter 63. The contact coefficient of a voxel separated by $G_D$ or more from the distal end 63d of the catheter 63 is 0. The contact coefficient of a voxel located at a center of the contact portion 64 is 1.

When Vp is visible voxel information, $\text{Color}_{CP}$ is a color of Vp, $\text{Color}_{Pd}$ is the predetermined color, and $\text{Color}_{LT}$ is a color of the biological tissue 60, the control unit 41 sets the color of each voxel of the three-dimensional image 53 by the following calculation.

$$\text{Color}_{CP} = C_F \times \text{Color}_{Pd} + (1 - C_F) \times \text{Color}_{LT}$$

Here, the term "visible voxel information" refers to voxel information in which the biological tissue 60 is present in the three-dimensional data 52. $\text{Color}_{CP}$, $\text{Color}_{Pd}$, and $\text{Color}_{LT}$ are RGB values. $\text{Color}_{Pd}$ is, for example, red. $\text{Color}_{LT}$ may be any color other than $\text{Color}_{Pd}$. $\text{Color}_{LT}$ may be a single color, or may be a color depending on a distance from a reference point, a reference line, or a reference plane. The reference point, the reference line, and the reference plane may be arranged at any position, but are preferably arranged according to the position of the opening 62. In the example of FIG. 5, the control unit 41 can arrange the reference point on a straight line in the cross section of the biological tissue 60 that passes through the position of the virtual camera 71, which is the viewpoint when the three-dimensional image 53 is displayed on the display 16, and the midpoint Pc of the third straight line L3 connecting the first end edge E1 of the opening 62 and the second end edge E2 of the opening 62. Therefore, the positions of the viewpoint, the reference point, and the midpoint Pc in a left-right direction are aligned, so that when the user observes the inner wall surface 61 of the biological tissue 60 through the opening 62, the user can easily grasp an unevenness and a depth by the difference in color tone of the three-dimensional image 53. In particular, when the reference point is arranged on the fourth straight line L4, the user can easily grasp the point Pt of the inner wall surface 61 of the biological tissue 60 and the unevenness and the depth around the point Pt. Since the $\text{Color}_{CP}$ is determined using the above-described equation, in the three-dimensional image 53, a color of a portion of the biological tissue 60 that is separated from the distal end 63d of the catheter 63 by $G_D$ or more coincides with $\text{color}_{LT}$. For convenience, in order to show the distal end 63d of the catheter 63, each point Pn of the inner wall surface 61 of the biological tissue 60, and an outer edge of the contact portion 64 of the biological tissue 60 in FIG. 9, grids at the corresponding positions are filled. By calculating $\text{Color}_{CP}$ using the above equation, the gradation is colored radially toward each grid indicated by 64, centering on the grid indicated by 63d.

Instead of calculating the distances from the distal end 63d of the catheter 63 to all the points of the biological tissue 60 in the three-dimensional data 52, the control unit 41 may calculate a distance from the distal end 63d of the catheter 63 to each point of the biological tissue 60 within a certain range including the contact point Pi in the three-dimensional data 52. In this case, the control unit 41 adjusts a ratio for including the element of the predetermined color in the color of each voxel of the voxel group of the three-dimensional image 53 corresponding to the range in accordance with the calculated distance. As an example, the control unit 41 may specify a calculation region in a certain range after specifying the contact point Pi, and calculate the distance Dist between the distal end 63d of the catheter 63 and each point Pn of the inner wall surface 61 of the biological tissue 60 only in the calculation region. According to this example, since it is not necessary to calculate the distance Dist for all voxels, the processing becomes faster. The contact portion 64 may be regarded as the "certain range". That is, in the three-dimensional image 53, gradation coloring may be performed only on the voxel group corresponding to the contact portion 64.

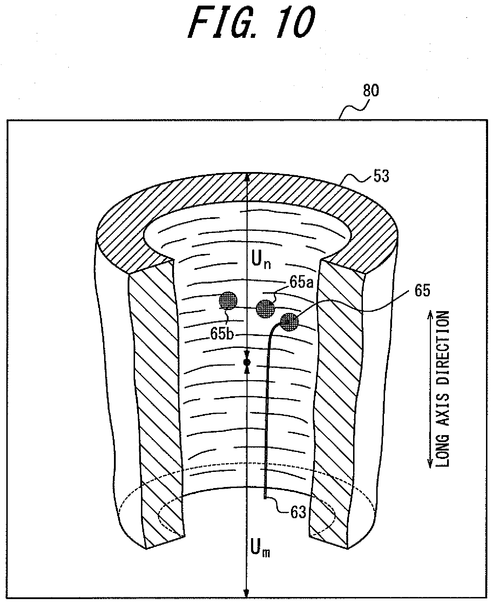

In a modification of the present embodiment, as shown in FIG. 10, when the control unit 41 receives input of cauterization data indicating that a partial region of the biological tissue 60 including the contact point Pi is cauterized by the ablation catheter as the catheter 63, the control unit 41 may specify the region as a cauterization region 65. The control unit 41 may set a color of a voxel group corresponding to the cauterization region 65 to a color different from the predetermined color in the three-dimensional image 53 displayed in step S3. The term "color different from the predetermined color" is orange in the modification, but may be any color as long as the voxel group corresponding to the cauterization region 65 can be distinguished from the voxel group corresponding to the contact portion 64 and the other voxel groups. In the example of FIG. 10, the cauterization data is input a plurality of times. Therefore, similarly to the voxel group corresponding to the cauterization region 65, the colors of the voxel groups corresponding to the other cauterization regions 65a and 65b are also set to orange.

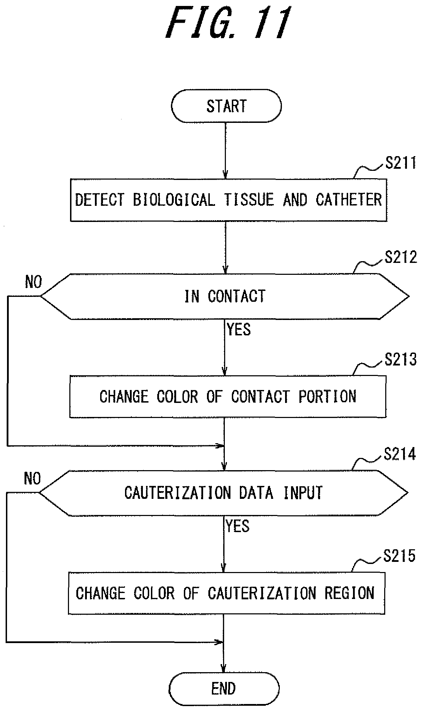

The details of the processing performed in step S2 when the ablation catheter is inserted into the biological tissue 60 and the ablation procedure is performed in the modification will be described with reference to FIG. 11.

Since the processing from step S211 to step S213 is the same as the processing from step S201 to step S203 in FIG. 7, the description thereof will be omitted.

When the cauterization data is input in step S214, the control unit 41 of the diagnostic assistance device 11 changes a color tone of the cauterization region 65 including the center of the contact portion 64. A size of the cauterization region 65 may be the same as that of the contact portion 64, or may be slightly larger or slightly smaller. Any method may be used as a method for receiving input of the cauterization data, and the modification uses a method of receiving, via the input unit 44, an input operation such as an operation of clicking a corresponding icon or an operation of pressing a corresponding shortcut key by the operator when the biological tissue 60 and the distal end of the catheter 63 are in contact with each other. Alternatively, a method of acquiring, via the communication unit 43, application information from a device that controls energy application or voltage application to the ablation catheter when the biological tissue 60 and the distal end of the catheter 63 are in contact with each other may be used.

When no cauterization data is input in step S214, the processing of step S215 is skipped.

As described above, in the present embodiment, the control unit 41 of the diagnostic assistance device 11 generates the three-dimensional data 52 of the biological tissue 60 inserted with the catheter 63 based on the tomographic data 51 of the biological tissue 60. The control unit 41 displays the generated three-dimensional data 52 as the three-dimensional image 53 on the display 16. In the three-dimensional data 52, the control unit 41 specifies the point of the biological tissue 60 with which the distal end of the catheter 63 is in contact as the contact point Pi. The control unit 41 sets the color of the voxel corresponding to the contact point Pi to the predetermined color in the three-dimensional image 53.

The present embodiment facilitates understanding of whether the catheter 63 and the biological tissue 60 are in contact with each other in the three-dimensional image 53. For example, when the user is an operator who performs an ablation procedure, it is easy to understand whether the ablation catheter and the tissue in a cardiac cavity are in contact with each other, and thus it is easy to perform the ablation procedure.

In the present embodiment, when the catheter 63 and the wall of the biological tissue 60 are in contact with each other, the contact between the catheter 63 and the wall of the biological tissue 60 can be indicated in the three-dimensional space by changing the color of the contact portion 64.

In the present embodiment, once the position of the opening 62 is determined, the positions of the camera 71 and the light sources 72 move such that the inside of the biological tissue 60 can be seen from the opening 62. Therefore, when the position of the opening 62 is changed to another position, it is possible to avoid a situation that only the outer wall surface of the biological tissue 60 can be seen and an object of interest cannot be confirmed.

The present disclosure is not limited to the above-described embodiment. For example, a plurality of blocks described in a block diagram may be integrated, or one block may be divided. Instead of executing a plurality of steps described in a flowchart in time series according to the description, the steps may be executed in parallel or in a different order according to the processing capability of the device that executes each step or as necessary. In addition, modifications can be made without departing from a gist of the present disclosure.

Reference Sign List

10 diagnostic assistance system
11 diagnostic assistance device
12 cable
13 drive unit

| | |
|---|---|
| 14 | keyboard |
| 15 | mouse |
| 16 | display |
| 17 | connection terminal |
| 18 | cart unit |
| 20 | probe |
| 21 | drive shaft |
| 22 | hub |
| 23 | sheath |
| 24 | outer tube |
| 25 | ultrasound transducer |
| 26 | relay connector |
| 31 | scanner unit |
| 32 | slide unit |
| 33 | bottom cover |
| 34 | probe connection unit |
| 35 | scanner motor |
| 36 | insertion port |
| 37 | probe clamp unit |
| 38 | slide motor |
| 39 | switch group |
| 41 | control unit |
| 42 | storage unit |
| 43 | communication unit |
| 44 | input unit |
| 45 | output unit |
| 51 | tomographic data |
| 52 | three-dimensional data |
| 53 | three-dimensional image |
| 60 | biological tissue |
| 61 | inner wall surface |
| 62 | opening |
| 63 | catheter |
| 63d | distal end |
| 64 | contact portion |
| 65 | cauterization region |
| 65a | cauterization region |
| 65b | cauterization region |
| 71 | camera |
| 72 | light source |
| 80 | screen |

**Claims**

1. A diagnostic assistance device configured to generate three-dimensional data of a biological tissue inserted with a catheter based on tomographic data of the biological tissue, and display the generated three-dimensional data as a three-dimensional image on a display, the diagnostic assistance device comprising:
a control unit configured to specify a point of the biological tissue with which a distal end of the catheter is in contact as a contact point in the three-dimensional data, and set a color of a voxel corresponding to the contact point to a predetermined color in the three-dimensional image.

2. The diagnostic assistance device according to claim 1, wherein
the control unit is configured to adjust a ratio for including an element of the predetermined color in a color of each voxel of the three-dimensional image in accordance with a distance from the distal end of the catheter to each point of the biological tissue in the three-dimensional data.

3. The diagnostic assistance device according to claim 1 or claim 2, wherein

EP 4 042 944 A1

the control unit is configured to analyze the tomographic data to detect a position of the biological tissue with which the distal end of the catheter is in contact, and specify a point corresponding to the detected position as the contact point in the three-dimensional data.

4. The diagnostic assistance device according to claim 1 or claim 2, wherein
the control unit is configured to analyze the three-dimensional data to specify the contact point.

5. The diagnostic assistance device according to claim 4, wherein
the control unit is configured to receive input of position data indicating a position of the biological tissue with which the distal end of the catheter is in contact, and correct an analysis result of the three-dimensional data with reference to the position data.

6. The diagnostic assistance device according to claim 1 or claim 2, wherein
the control unit is configured to receive input of position data indicating a position of the biological tissue with which the distal end of the catheter is in contact, and specify a point corresponding to the position indicated by the position data as the contact point in the three-dimensional data.

7. The diagnostic assistance device according to claim 1, wherein
the control unit is configured to calculate a distance from the distal end of the catheter to each point of the biological tissue within a certain range in the three-dimensional data including the contact point, and adjust a ratio for including an element of the predetermined color in a color of each voxel of a voxel group of the three-dimensional image corresponding to the range in accordance with the calculated distance.

8. The diagnostic assistance device according to any one of claims 1 to 7, wherein
when the control unit receives input of cauterization data indicating that a partial region of the biological tissue including the contact point is cauterized by an ablation catheter as the catheter, the control unit sets a color of a voxel group corresponding to the region to a color different from the predetermined color.

9. A diagnostic assistance system, comprising:

the diagnostic assistance device according to any one of claims 1 to 8; and
a sensor configured to acquire the tomographic data while moving in the biological tissue.

10. The diagnostic assistance system according to claim 9, further comprising the display.

11. A diagnostic assistance method for generating three-dimensional data of a biological tissue inserted with a catheter based on tomographic data of the biological tissue, and displaying the generated three-dimensional data as a three-dimensional image on a display, the diagnostic assistance method comprising:

specifying a point of the biological tissue with which a distal end of the catheter is in contact as a contact point in the three-dimensional data; and
setting a color of a voxel corresponding to the contact point to a predetermined color in the three-dimensional image.

# FIG. 1

FIG. 2

## *FIG. 3*

11

DIAGNOSTIC ASSISTANCE DEVICE

CONTROL UNIT — 41

~51 ~52 ~53

| TOMOGRAPHIC DATA | → | THREE-DIMENSIONAL DATA | → | THREE-DIMENSIONAL IMAGE |

42 — STORAGE UNIT        COMMUNICATION UNIT — 43

44 — INPUT UNIT        OUTPUT UNIT — 45

# *FIG. 4*

```
        ( START )
           │
           ▼
┌──────────────────────────────────┐
│    ACQUIRE TOMOGRAPHIC DATA       │──S1
└──────────────────────────────────┘
           │
           ▼
┌──────────────────────────────────┐
│  GENERATE THREE-DIMENSIONAL DATA  │──S2
└──────────────────────────────────┘
           │
           ▼
┌──────────────────────────────────┐
│  DISPLAY THREE-DIMENSIONAL IMAGE  │──S3
└──────────────────────────────────┘
           │
           ▼
                        S4
          ◇ OPERATED BY USER ◇
   NO ◄──┘            │
                     YES
           ▼
┌──────────────────────────────────┐
│      RECEIVE OPERATION OF USER    │──S5
└──────────────────────────────────┘
           │
           ▼
┌──────────────────────────────────┐
│   DETERMINE POSITION OF OPENING   │──S6
└──────────────────────────────────┘
           │
           ▼
┌──────────────────────────────────┐
│          FORM OPENING             │──S7
└──────────────────────────────────┘
           │
           ▼
┌──────────────────────────────────┐
│        ADJUST VIEWPOINT           │──S8
└──────────────────────────────────┘
           │
           ▼
                        S9
      ◇ TOMOGRAPHIC DATA UPDATED ◇── NO
                     │
                    YES
```

# FIG. 5

# FIG. 6

# FIG. 7

```
          ┌─────────────┐
          │    START    │
          └──────┬──────┘
                 │
                 ▼                              ⌇S201
  ┌──────────────────────────────────────────┐
  │ DETECT BIOLOGICAL TISSUE AND CATHETER     │
  └──────────────────┬───────────────────────┘
                     │
                     ▼                          ⌇S202
  NO  ╱─────────────────────────────────────────╲
  ◄───┤              IN CONTACT                   │
      ╲─────────────────────┬─────────────────────╱
                     │YES
                     ▼                          ⌇S203
  ┌──────────────────────────────────────────┐
  │     CHANGE COLOR OF CONTACT PORTION       │
  └──────────────────┬───────────────────────┘
                     │
                     ▼
          ┌─────────────┐
          │     END     │
          └─────────────┘
```

# FIG. 8

VOXEL

INSERT INTO CORRESPONDING
POSITION IN LONG
AXIS DIRECTION

| 0 | 32 | 35 | 65 | 45 |
|---|---|---|---|---|
| 0 | 0 | 45 | 58 | 50 |
| 0 | 0 | 42 | 54 | 55 |
| 78 | 80 | 75 | 88 | 100 |
| 95 | 145 | 126 | 160 | 208 |

BINARIZE →

| 0 | 1 | 1 | 1 | 1 |
|---|---|---|---|---|
| 0 | 0 | 1 | 1 | 1 |
| 0 | 0 | 1 | 1 | 1 |
| 1 | 1 | 1 | 1 | 1 |
| 1 | 1 | 1 | 1 | 1 |

DOWN-
SAMPLING →

| 0 | 1 | 1 |
|---|---|---|
| 0 | 1 | 1 |
| 1 | 1 | 1 |

EP 4 042 944 A1

## FIG. 9

# FIG. 10

# *FIG. 11*

```
        ( START )
            |
            v
    ┌───────────────────────────────────────┐  ⌐S211
    │ DETECT BIOLOGICAL TISSUE AND CATHETER  │
    └───────────────────────────────────────┘
            |
            v
  NO  ╱─────────────────────────────────╲      ⌐S212
 ◄────      IN CONTACT                    ►
      ╲─────────────────────────────────╱
            | YES
            v
    ┌───────────────────────────────┐          ⌐S213
    │ CHANGE COLOR OF CONTACT PORTION│
    └───────────────────────────────┘
            |
            v
  NO  ╱─────────────────────────────────╲      ⌐S214
 ◄────   CAUTERIZATION DATA INPUT         ►
      ╲─────────────────────────────────╱
            | YES
            v
    ┌─────────────────────────────────┐        ⌐S215
    │ CHANGE COLOR OF CAUTERIZATION REGION│
    └─────────────────────────────────┘
            |
            v
        ( END )
```

EP 4 042 944 A1

# INTERNATIONAL SEARCH REPORT

| International application No. |
| --- |
| PCT/JP2020/036452 |

**A. CLASSIFICATION OF SUBJECT MATTER**
A61B 8/12(2006.01)i
FI: A61B8/12
According to International Patent Classification (IPC) or to both national classification and IPC

**B. FIELDS SEARCHED**

Minimum documentation searched (classification system followed by classification symbols)
A61B5/055, A61B6/00-6/14, A61B8/00-8/15

| Documentation searched other than minimum documentation to the extent that such documents are included in the fields searched | |
| --- | --- |
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2008-220802 A (HITACHI MEDICAL CORPORATION) 25 September 2008 (2008-09-25) paragraphs [0021], [0025], [0034], [0036], [0038], [0042], fig. 1-3 | 1, 3, 4, 6, 8-11 |
| Y | JP 2013-31655 A (TOSHIBA CORP.) 14 February 2013 (2013-02-14) paragraphs [0014], [0015], [0021], [0022], [0027], [0042], [0044], [0057], [0059], [0061], [0062], fig. 1, 6-10 | 1, 3, 4, 6, 8-11 |
| A | JP 2010-88584 A (TOSHIBA CORP.) 22 April 2010 (2010-04-22) paragraphs [0011]-[0013], [0057], [0058], [0061], fig. 6, 7 | 2, 5, 7 |

☐ Further documents are listed in the continuation of Box C.   ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 24 November 2020 (24.11.2020) | 08 December 2020 (08.12.2020) |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**
Information on patent family members

International application no.

PCT/JP2020/036452

| Patent Documents referred in the Report | Publication Date | Patent Family | Publication Date |
|---|---|---|---|
| JP 2008-220802 A | 25 Sep. 2008 | (Family: none) | |
| JP 2013-31655 A | 14 Feb. 2013 | US 2013/0116550 A1 paragraphs [0025], [0026], [0032], [0033], [0038], [0053], [0055], [0068], [0070], [0072]-[0074], fig. 1, 6-10 CN 1031185g5 A | |
| JP 2010-88584 A | 22 Apr. 2010 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100215238
- US 6385332 B
- US 6251072 B